# EUROPÄISCHE PATENTSCHRIFT

(11) **EP 1 257 518 B1**
(45) Veröffentlichungstag und Bekanntmachung des Hinweises auf die Patenterteilung: **11.04.2012**
(21) Anmeldenummer: 01916952.3
(22) Anmeldetag: 26.01.2001
(51) Int. Cl.: C07C 29/141, C07C 2/10, C01B 3/34

(54) **VERFAHREN ZUR HERSTELLUNG VON C 9-ALKOHOLEN UND VERFAHREN ZUR INTEGRIERTEN HERSTELLUNG VON C 9-ALKOHOLEN UND C 10-ALKOHOLEN**
METHOD FOR PRODUCING C 9 ALCOHOLS AND METHOD FOR THE INTEGRATED PRODUCTION OF C 9 ALCOHOLS AND C 10 ALCOHOLS
PROCEDE DE PREPARATION D'ALCOOLS C 9 ET PROCEDE DE PREPARATION INTEGREE D'ALCOOLS C 9 ET D'ALCOOLS C 10

(30) Priorität: 27.01.2000 DE 10003482
(43) Veröffentlichungstag der Anmeldung: 20.11.2002
(73) Patentinhaber: BASF SE, 67056 Ludwigshafen (DE)
(72) Erfinder: MCATEE, Michael, Richard, Jackson, TX 77566 (US); PACIELLO, Rocco, 67098 Bad Dürkheim (DE); ULBRICH, Michael-Dieter, 67251 Freinsheim (DE)
(86) Internationale Anmeldenummer: PCT/EP2001/000854
(87) Internationale Veröffentlichungsnummer: WO 2001/055065

(56) Entgegenhaltungen:
- WO-A-95/14647
- WO-A-99/25668
- DE-A- 2 316 084
- PATENT ABSTRACTS OF JAPAN vol. 015, no. 181 (C-0830), 9. Mai 1991 (1991-05-09) & JP 03 044340 A (MITSUBISHI KASEI CORP), 26. Februar 1991 (1991-02-26)
- PATENT ABSTRACTS OF JAPAN vol. 016, no. 207 (C-0941), 18. Mai 1992 (1992-05-18) & JP 04 036251 A (MITSUBISHI KASEI CORP), 6. Februar 1992 (1992-02-06)

## Beschreibung

Die vorliegende Erfindung betrifft ein Verfahren zur Herstellung von C₉-Alkoholen und außerdem ein Verfahren zur integrierten Herstellung von C₉-Alkoholen und C₁₀-Alkoholen.

Fossile Brennstoffe und die daraus gewonnenen Kohlenwasserstoffe haben in der industriellen Synthese eine Doppelfunktion. Sie dienen zum einen als Energieträger und zum anderen als Rohstoffe für chemische Produkte. Es ist vielfach üblich, Kohlenwasserstoffe, die bei der industriellen Synthese als Nebenprodukte anfallen oder an spezifischen Wertprodukten verarmt sind, zu verbrennen, um den darin enthaltenen Energiegehalt verfügbar zu machen. Es besteht gegenwärtig eine Tendenz, fossile Rohstoffe auf dem Energiesektor zu substituieren und damit die Rohstoffversorgung bei einem gegebenen Angebot an fossilen Rohstoffen langfristig zu sichern. Hierzu ist es erforderlich, die in den aus fossilen Rohstoffen gewonnenen Kohlenwasserstoffen enthaltenen Komponenten möglichst weitgehend stofflich zu nutzen. Nachteiligerweise fallen bei der industriellen Synthese Kohlenwasserstoffströme meist als schlecht definierte Gemische wechselnder Zusammensetzung an. Die stoffliche Verwertung der darin enthaltenen Wertkomponenten scheitert oft an dem unverhältnismäßig hohen Aufwand der Reinigung bzw. Auftrennung. Es gilt, Verfahrensverbunde anzugeben, bei denen eine stoffliche Nutzung der Nebenprodukte einer Verfahrensstufe in einer weiteren Verfahrensstufe ohne das Erfordernis aufwendiger Trennung möglich ist.

Es ist bekannt, C₄-Schnitte, die in großen Mengen sowohl aus FCC-Anlagen als auch aus Steamcrackern zur Verfügung stehen und die im Wesentlichen aus einem Gemisch von Buten und Butan bestehen, zur Herstellung von Buten-Oligomeren, insbesondere Octenen, einer Oligomerisierungsreaktion zu unterwerfen. Ein derartiges Verfahren ist z. B. in der DE 4 339 713 beschrieben. Bei dieser Umsetzung fällt neben den Buten-Oligomeren ein Buten-abgereicherter C₄-Kohlenwasserstoffstrom an, in dem die Butene soweit durch Butan verdünnt sind, dass die Oligomerisierung der darin noch enthaltenen Butene nicht mehr praktisch sinnvoll betrieben werden kann. Die Octene werden üblicherweise zu C₉-Aldehyden hydroformyliert und weiter zu C₉-Alkoholen hydriert. Die C₉-Alkohole weisen wertvolle Eigenschaften als Weichmacher-Alkohole auf.

Es ist ferner bekannt, Synthesegas durch Steamreforming oder partielle Oxidation von Kohlenwasserstoffen herzustellen (vgl. Weissermel, K. und Arpe H.-J., "Industrielle organische Chemie", VCH, 4. Aufl. 1994, Seite 19-24.

Gusev I. N. et al. berichten in Sb. Nauchn. Tr.- Vses. Nauchno-Issled Inst. Pererab. Nefti (1981), 39, 11-20 von der Herstellung von Wasserstoff durch katalytisches Steamreforming von Raffineriegasen, die Wasserstoff, C₁₋₄-Alkane und etwa 20 % Alkene enthalten. Eine vorgeschaltete Hydrierung wird als vorteilhaft beschrieben.

Die JP 52132-004 beschreibt die Herstellung eines 50 bis 95 Mol-% H₂, 1 bis 50 Mol-% CO, < 25 Mol-% CO₂ und < 25 Mol-% Methan enthaltenden Gases durch Behandeln eines Kohlenwasserstoffs mit einem NiO-Katalysator in Gegenwart von Dampf.

Yokoyama, A. berichtet in Nippon Gasu Kyokaishi (1978), 31(8), 21-9 von Ergebnissen bei der katalytischen Reformierung von C₄-Alkanen und einer Fraktion mit hohem Gehalt an C₄-Alkanen und -Alkenen.

Der vorliegenden Erfindung liegt die Aufgabe zugrunde, ein von C₄-Kohlenwasserstoffen ausgehendes Verfahren zur Herstellung von C₉-Alkoholen bzw. ein Verfahren zur Herstellung von C₉-Alkoholen und C₁₀-Alkoholen bereitzustellen, das eine möglichst weitgehende stoffliche Nutzung der Einsatz-Kohlenwasserstoffe erlaubt.

Es wurde nun gefunden, dass der bei der Buten-Oligomerisierung anfallende Buten-abgereicherte C₄-Kohlenwasserstoffstrom mit Vorteil als Ausgangsstoff für die Herstellung von Synthesegas verwendet werden kann, welches zur Hydroformylierung der durch Butendimerisierung erhaltenen Octene zu C₉-Aldehyden und/oder von Buten zu C₅-Aldehyden herangezogen werden kann.

Die Erfindung betrifft daher in einem ersten Aspekt ein Verfahren zur Herstellung von C₉-Alkoholen, bei dem man
a) einen Buten und Butan enthaltenden C₄-Kohlenwasserstoffstrom bereitstellt;
b) den C₄-Kohlenwasserstoffstrom einer Oligomerisierung an einem Olefin-Oligomerisierungskatalysator unterwirft und das erhaltene Reaktionsgemisch auftrennt, wobei ein Octene enthaltender Strom und ein Buten-abgereicherter C₄-Kohlenwasserstoffstrom erhalten werden;
c) den Buten-abgereicherten C₄-Kohlenwasserstoffstrom einem Steamreforming oder einer partiellen Oxidation unterwirft, wobei Kohlenmonoxid und Wasserstoff erhalten werden;
d) den Octene enthaltenden Strom in Gegenwart eines Hydroformylierungskatalysators mit Kohlenmonoxid und Wasserstoff zu C₉-Aldehyden hydroformyliert, wobei das eingesetzte Kohlenmonoxid und der eingesetzte Wasserstoff zu mehr als 80 %, insbesondere vollständig aus dem Schritt c) stammen; und
e) die C₉-Aldehyde mit Wasserstoff katalytisch hydriert, wobei der in Schritt e) eingesetzte Wasserstoff zu mehr als 80 %, insbesondere vollständig, aus dem Schritt c) stammt.

Der "Buten-abgereicherte C₄-Kohlenwasserstoffstrom" ist gegenüber dem eingesetzten C₄-Kohlenwasserstoffstrom an Buten verarmt. Die Menge der enthaltenen Butene ist um einen Wert verringert, die dem Buten-Umsatz der Oligomerisierung entspricht. Im Allgemeinen ist der Buten-Gehalt des Buten-abgereicherten C₄-Kohlenwasserstoffstroms gegenüber dem des eingesetzten C₄-Kohlenwasserstoffstroms um 70 bis 99 %, meist 80 bis 95 % verringert. Der Buten-abgereicherte C₄-Kohlenwasserstoffstrom enthält z. B. 5 bis 70 Mol-%, meist 5 bis 45 Mol-% Buten, wobei der Rest im Wesentlichen aus Butan besteht.

Die Erfindung betrifft in einem zweiten Aspekt außerdem ein Verfahren zur integrierten Herstellung von C₉-Alkoholen und C₁₀-Alkoholen, bei dem man
a) einen Buten und Butan enthaltenden C₄-Kohlenwasserstoffstrom bereitstellt;
b) den C₄-Kohlenwasserstoffstrom in Gegenwart eines Hydroformylierungskatalysators mit Kohlenmonoxid und Wasserstoff zu C₅-Aldehyden hydroformyliert, wobei ein erster Buten-abgereicherter C₄-Kohlenwasserstoffstrom erhalten wird;
c) die C₅-Aldehyde einer Aldolkondensation unterwirft; und
d) die Produkte der Aldolkondensation mit Wasserstoff katalytisch zu C₁₀-Alkoholen hydriert;
e) den ersten Buten-abgereicherten C₄-Kohlenwasserstoffstrom an einem Olefin-Oligomerisierungskatalysator einer Oligomerisierung unterwirft und das erhaltene Reaktionsgemisch auftrennt, wobei ein Octene enthaltender Strom und ein zweiter Buten-abgereicherter C₄-Kohlenwasserstoffstrom erhalten werden;
f) den Octen enthaltenden Strom in Gegenwart eines Hydroformylierungskatalysators mit Kohlenmonoxid und Wasserstoff zu C₉-Aldehyden hydroformyliert;
g) die C₉-Aldehyde mit Wasserstoff katalytisch hydriert;
h) den zweiten Buten-abgereicherten C₄-Kohlenwasserstoffstrom einem Steamreforming oder einer partiellen Oxidation unterwirft, wobei Kohlenmonoxid und Wasserstoff erhalten werden, welche zumindest teilweise in Schritt b) und Schritt f) zurückgeführt werden, wobei der in Schritt d) und in Schritt g) eingesetzte Wasserstoff zu mehr als 80 %, insbesondere vollständig, aus Schritt h) Stammt.

Der "erste Buten-abgereicherte C₄-Kohlenwasserstoffstrom" ist gegenüber dem eingesetzten C₄-Kohlenwasserstoffstrom, der "zweite Buten-abgereicherte C₄-Kohlenwasserstoffstrom" gegenüber dem ersten Buten-abgereicherten C₄-Kohlenwasserstoffstrom an Buten verarmt. Im Allgemeinen ist der Buten-Gehalt des ersten Buten-abgereicherten C₄-Kohlenwasserstoffstroms um 25 bis 50 %, der des zweiten Buten-abgereicherten C₄-Kohlenwasserstoffstroms gegenüber dem des ersten Buten-abgereicherten C₄-Kohlenwasserstoffstroms um 70 bis 99 % verringert. Der erste Buten-abgereicherte C₄-Kohlenwasserstoffstrom enthält z. B. 30 bis 60 Mol-%, der zweite Buten-abgereicherte C₄-Kohlenwasserstoffstrom z. B. 0,3 bis 20 Mol-% Buten, wobei der Rest im Wesentlichen aus Butan besteht.

Als Einsatzmaterial geeignete C₄-Kohlenwasserstoffströme enthalten z. B. 50 bis 99, vorzugsweise 60 bis 90 Mol-% Butene und 1 bis 50, vorzugsweise 10 bis 40 Mol-% Butane. Vorzugsweise umfasst die Butenfraktion 40 bis 60 Mol-% 1-Buten, 20 bis 30 Mol-% 2-Buten und weniger als 5 Mol-%, insbesondere weniger als 3 Mol-% Isobuten (bezogen auf die Butenfraktion). Als besonders bevorzugter Einsatzstoff wird das sogenannte Raffinat II verwendet, bei dem es sich um ein Isobuten-abgereicherten C₄-Schnitt aus einer FCC-Anlage oder einen Steamcracker handelt. Raffinat II weist folgende typische Zusammensetzung auf:

| | |
|---|---|
| i-, n-Butan | 26 Mol-% |
| i-Buten | 1 Mol-% |
| 1-Buten | 26 Mol-% |
| trans-2-Buten | 31 Mol-% |
| cis-2-Buten | 16 Mol-% |

Sind Diolefine oder Alkine im C₄-Kohlenwasserstoffstrom vorhanden, so werden diese vor der Oligomerisierung vorzugsweise auf weniger als 10 ppm, insbesondere auf weniger als 5 ppm, entfernt. Sie werden bevorzugt durch selektive Hydrierung, z. B. gemäß EP-81 041 und DE-1 568 542, entfernt. Vorzugsweise werden außerdem sauerstoffhaltige Verbindungen, wie Alkohole, Aldehyde, Ketone oder Ether weitgehend entfernt. Hierzu kann der C₄-Kohlenwasserstoffstrom mit Vorteil über ein Adsorptionsmittel, wie z. B. ein Molekularsieb, insbesondere eines mit einem Porendurchmesser von > 4 Å bis 5 Å, geleitet werden.

Die einzelnen Stufen der erfindungsgemäßen Verfahren sind an sich bekannt und in ihrer spezifischen Ausgestaltung nicht Gegenstand der vorliegenden Erfindung. Die einzelnen Stufen werden im Folgenden anhand exemplarischer oder bevorzugter Ausführungsformen näher erläutert.

### Oligomerisierung

Zur Oligomerisierung, insbesondere Dimerisierung, niederer Olefine, wie Butenen, ist eine Reihe von Verfahren bekannt. Jedes der bekannten Verfahren ist prinzipiell zur Durchführung der Buten-Oligomerisierungsstufe der erfindungsgemäßen Verfahren geeignet.

Die Oligomerisierung von Olefinen kann unter homogener oder heterogener Katalyse erfolgen. Ein Beispiel eines homogen katalysierten Verfahrens ist der DIMERSOL-Prozess. Beim DIMERSOL-Prozess (vergleiche Revue de l'Institut Français du Petrol, Vol. 37, No. 5, Sept./Okt. 1982, Seite 639ff) werden niedere Olefine in flüssiger Phase dimerisiert. Als Vorläufer der katalytisch aktiven Spezies sind z. B. (i) das System π-Allyl-Nickel/Phosphin/Aluminiumhalogenid, (ii) Ni(O)-Verbindungen in Kombination mit LewisSäuren, wie Ni(COD)₂ + AXₙ oder Ni(CO)₂(PR₃) + AXₙ, oder (iii) Ni(II)-Komplexe in Kombination mit Alkylaluminiumhalogeniden, wie NiX₂(PR₃)₂ + Al₂Et₃Cl₃ oder Ni(OCOR)₂ + AlEtCl₂, geeignet (mit COD = 1,5-Cyclooctadien, X = Cl, Br, I; R = Alkyl, Phenyl; AXₙ = AlCl₃, BF₃, SbF₅ etc.). Nachteilig bei den homogen katalysierten Verfahren ist die aufwendige Katalysatorabtrennung.

Diese Nachteile bestehen bei den heterogen katalysierten Verfahren nicht. Bei diesen Verfahren wird in der Regel ein olefinhaltiger Strom bei erhöhter Temperatur über den fest angeordneten heterogenen Katalysator geleitet.

Technisch weit verbreitet ist das Verfahren der UOP unter Verwendung von H₃PO₄/SiO₂ in einem Festbett (vergleiche z. B. US 4,209,652, US 4,229,586, US 4,393,259). Beim Bayer-Verfahren werden saure Ionenaustauscher als Katalysator eingesetzt (vergleiche z. B. DE 195 35 503, EP-48 893). Die WO 96/24567 (Exxon) beschreibt den Einsatz von Zeolithen als Oligomerisierungskatalysatoren. Ionenaustauscher wie Amberlite werden auch beim Verfahren der Texas Petro Chemicals (vergleiche DE 3 140 153) eingesetzt.

Es ist weiter bekannt, niedere Olefine unter Alkalimetall-Katalyse zu dimerisieren (vergleiche Catalysis Today, 1990, 6, S. 329ff).

Für die vorliegenden Zwecke ist es bevorzugt, die Buten-Oligomerisierung an einem heterogenen Nickel enthaltenden Katalysator durchzuführen. Die verwendbaren heterogenen, Nickel enthaltenden Katalysatoren können unterschiedliche Struktur aufweisen, wobei Nickeloxid enthaltende Katalysatoren bevorzugt sind. Es kommen an sich bekannte Katalysatoren in Betracht, wie sie in C.T. O'Connor et al., Catalysis Today, Band 6 (1990), Seite 336-338 beschrieben sind. Insbesondere werden trägergebundene Nickelkatalysatoren eingesetzt. Die Trägermaterialien können z. B. Kieselsäure, Tonerde, Alumosilikate, Alumosilikate mit Schichtstrukturen und Zeolithe, Zirkoniumoxid, das gegebenenfalls mit Säuren behandelt ist, oder sulfatiertes Titandioxid sein. Besonders geeignet sind Fällungskatalysatoren, die durch Mischen wässriger Lösungen von Nickelsalzen und Silikaten, z. B. Natriumsilikat mit Nickelnitrat, und gegebenenfalls Aluminiumsalzen, wie Aluminiumnitrat und Calcinieren erhältlich sind. Weiterhin sind Katalysatoren verwendbar, die durch Einlagerung von Ni²⁺-Ionen durch Ionenaustausch in natürliche oder synthetische Schichtsilikate, wie Montmorillonite, erhalten werden. Geeignete Katalysatoren können auch durch Imprägnieren von Kieselsäure, Tonerde oder Alumosilikaten mit wässrigen Lösungen löslicher Nickelsalze, wie Nickelnitrat, Nickelsulfat oder Nickelchlorid, und anschließende Calcinierung erhalten werden.

Besonders bevorzugt sind Katalysatoren, die im Wesentlichen aus NiO, SiO₂, TiO₂ und/oder ZrO₂ sowie gegebenenfalls Al₂O₃ bestehen. Sie führen zu einer Bevorzugung der Dimerisierung gegenüber der Bildung höherer Oligomere und liefern überwiegend lineare Produkte. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE 4 339 713 verwiesen. Auf die Offenbarung dieser Druckschrift und den darin zitierten Stand der Technik wird vollinhaltlich Bezug genommen.

Der Katalysator liegt vorzugsweise in stückiger Form, z. B. in Form von Tabletten, z. B. mit einem Durchmesser von 2 bis 6 mm und einer Höhe von 3 bis 5 mm, Ringen mit z. B. 5 bis 7 mm Außendurchmesser, 2 bis 5 mm Höhe und 2 bis 3 mm Lochdurchmesser oder Strängen unterschiedlicher Länge eines Durchmessers von z. B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Die Oligomerisierung am heterogenen, Nickel enthaltenden Katalysator erfolgt vorzugsweise bei Temperaturen von 30 bis 280 °C, insbesondere 30 bis 140 °C und besonders bevorzugt von 40 bis 130 °C. Sie erfolgt vorzugsweise bei einem Druck von 10 bis 300 bar, insbesondere von 15 bis 100 bar und besonders bevorzugt von 20 bis 80 bar. Der Druck wird dabei zweckmäßigerweise so eingestellt, dass bei der gewählten Temperatur der C₄-Kohlenwasserstoffstrom flüssig oder im überkritischen Zustand vorliegt.

Der C₄-Kohlenwasserstoffstrom wird zweckmäßigerweise über ein oder mehrere fest angeordnete Katalysatoren geleitet. Geeignete Reaktionsapparaturen für das Inkontaktbringen des C₄-Kohlenwasserstoffstroms mit dem heterogenen Katalysator sind dem Fachmann bekannt. Geeignet sind z. B. Rohrbündelreaktoren oder Schachtöfen. Aufgrund der geringeren Investitionskosten sind Schachtöfen bevorzugt. Die Oligomerisierung kann in einem einzelnen Reaktor durchgeführt werden, wobei der Oligomerisierungskatalysator in einem einzigen oder mehreren Festbetten im Reaktor angeordnet sein kann. Alternativ kann zur Durchführung der Oligomerisierung eine Reaktorkaskade aus mehreren, vorzugsweise 2, hintereinander geschalteten Reaktoren eingesetzt werden, wobei beim Passieren des bzw. der dem letzten Reaktor der Kaskade vorgeschalteten Reaktors bzw. Reaktoren die Oligomerisierung im C₄-Kohlenwasserstoffstrom nur bis zu einem Teilumsatz betrieben wird und der gewünschte Endumsatz erst beim Passieren des Reaktionsgemisches durch den letzten Reaktor der Kaskade erzielt wird.

Nach dem Verlassen des Reaktors bzw. des letzten Reaktors einer Kaskade wird das Reaktionsgemisch aufgetrennt in einen Octene enthaltenden Strom, gegebenenfalls höhere Oligomere und in einen Buten-abgereicherten C₄-Kohlenwasserstoffstrom. Der Octene enthaltende Strom besteht vorzugsweise zu mehr als 97 Gew.-%, insbesondere im Wesentlichen vollständig, aus isomeren Octenen.

Die obigen Ausführungen zur Olefin-Oligomerisierung gelten entsprechend für den Einsatz des ersten Buten-abgereicherten C₄-Kohlenwasserstoffstroms gemäß dem zweiten Aspekt der Erfindung.

### Steamreforming

Beim Steamreforming (allotherme Dampfspaltung) wird in Gegenwart von H₂O eine katalytische Spaltung von Kohlenwasserstoffen vorgenommen, wobei ein Gemisch von Kohlenmonoxid und Wasserstoff, das sogenannte Synthesegas, erhalten wird. Der Wärmebedarf für die endotherme Umsetzung wird von außen gedeckt. Die Stoffumwandlung bei dem Steamreforming lässt sich durch die folgende Bruttoformel beschreiben:

CHₓ + H₂O → CO + H₂ + x/2 H₂

Aus dieser Gleichung ist ersichtlich, dass der Beitrag des Kohlenwasserstoff-Einsatzmaterials zum Anteil an gebildetem H₂ mit dessen Wasserstoffgehalt zunimmt. Ein im erfindungsgemäßen Verfahren anfallender Buten-abgereicherter C₄-Kohlenwasserstoffstrom weist z. B. eine typische Zusammensetzung von 19 Mol-% Buten und 81 Mol-% Butan auf. Der Buten-abgereicherte C₄-Kohlenwasserstoffstrom weist in diesem Fall eine durchschnittliche Summenformel von C₄H_{9,62} auf:

C₄H_{9,62} + 4 H₂O → 4 CO + 8,81 H₂

Das molare H₂/CO-Verhältnis des erhaltenen Synthesegases beträgt somit 2,2:1.

Der Hydroformylierungsschritt der erfindungsgemäßen Verfahren, der weiter unten näher beschrieben wird, lässt sich durch folgende Nettoformel beschreiben:

C₈H₁₆ + CO + H₂ → C₈H₁₇-CHO

der Hydrierungsschritt durch folgende Nettoformel:

C₈H₁₇-CHO + H₂ → C₈H₁₇-CH₂OH

Man sieht, dass für die Kombination der Schritte der Hydroformylierung und Hydrierung Wasserstoff und Kohlenmonoxid in einem molaren Verhältnis von 2:1 benötigt werden. Wie oben gezeigt, weist das durch Steamreforming des Buten-abgereicherten C₄-Kohlenwasserstoffstroms erhaltene Synthesegas ein gut passendes H₂:CO-Molverhältnis auf.

Die Verfahrensweise beim Steamreforming besteht in der Regel darin, dass eine Mischung von Kohlenwasserstoff und Wasserdampf in von außen beheizten Rohren an geeigneten Katalysatoren, z. B. Nickelkatalysatoren, bei Normaldruck oder erhöhtem Druck und bei Temperaturen von z. B. 700 bis 950 °C kontinuierlich umgesetzt wird. Eine Vielzahl parallel geschalteter, mit Katalysator beschickter Rohre, meist aus Chrom-Nickel-Stahl, ist vertikal in feuerfest ausgemauerten Reaktoren derart eingebaut, dass sie sich frei ausdehnen können. Der Rohrdurchmesser beträgt beispielsweise 15 bis 20 cm, die beheizte Rohrlänge 9 m. Durch Brenner werden die Rohre von außen beheizt. Zur Beheizung der Rohre kann ein externes Brennmittel oder vorzugsweise eine Teilmenge des Buten-abgereicherten C₄-Kohlenwasserstoffs verwendet werden. Zur Herstellung methanarmer Synthesegase kann man das Gas aus dem Röhrenofen in einen als Schachtofen ausgebildeten Nachverbrennungsofen leiten.

Geeignete Katalysatoren für das Steamreforming sind z. B. in Max Appl, Modern Production Technologies, published by Nitrogen, British Sulphur Publishing, 1997, S. 10ff, beschrieben.

Mit Vorteil kann man das als Nebenprodukt gebildete CO₂ abtrennen (siehe unten) und in den Steamreformer ganz oder teilweise zurückführen. Das zurückgeführte Kohlendioxid reagiert mit den Kohlenwasserstoffen CHₓ unter Bildung von nutzbarem Wasserstoff und Kohlenmonoxid.

In einer bevorzugten Ausführungsform umfasst der Steamreformierungsschritt eine teilweise oder vollständige Hydrierung des Buten-abgereicherten C₄-Kohlenwasserstoffstroms, um das noch enthaltene Buten ganz oder teilweise in Butan umzuwandeln, bevor der C₄-Kohlenwasserstoffstrom in den Reformer geleitet wird. Der Buten-Gehalt des Stroms wird dabei vorzugsweise auf weniger als 5 Mol-%, insbesondere auf weniger als 1 Mol-% verringert. Durch die Hydrierung wird die Gefahr einer Kohlenstoff-Abscheidung auf dem Reformierungskatalysator, die bei hohen Butenrestgehalten bzw. hohen Katalysatorbelastungen möglich ist, vermieden. Die Kohlenstoff-Abscheidung kann zwar auch durch ein höheres Wasserdampf-Kohlenwasserstoff-Verhältnis verringert werden, doch geht dies auf Kosten des thermischen Wirkungsgrades und des Kohlenmonoxidgehalts im Endgas. Zur vorgeschalteten Hydrierung des Buten-abgereicherten C₄-Kohlenwasserstoffstroms wird mit Vorteil rückgeführtes Wasserstoffgas herangezogen. Die Isolierung von Wasserstoff aus dem beim Steamreforming gebildeten Synthesegas kann z. B. durch Druckwechseladsorption oder Tieftemperaturdestillation vorgenommen werden. Derartige Verfahren sind z. B. bei Max Appl, loc. cit., S. 108ff beschrieben.Geeignete Anlagen zur Synthesegasherstellung durch Steamreforming sind z. B. in Max Appl, loc. cit., S. 7ff, beschrieben.

Geeignete Katalysatoren zur vollständigen oder teilweisen Hydrierung des Buten-abgereicherten C₄-Kohlenwasserstoffstroms sind z. B. Nickeloxid, Kobaltoxid und/oder Molybdänoxid auf Aluminiumoxid-enthaltenden Trägermaterialien.

In einer bevorzugten Ausführungsform wird vor dem Steamreforming bzw. zwischen der Hydrierung und dem Steamreforming ein Prereforming-Schritt durchgeführt. Der Prereformer arbeitet bei niedrigerer Temperatur als der eigentliche Steamreformer. Typische Betriebstemperaturen des Prereformers sind 400 bis 550 °C. Die Mischung von Kohlenwasserstoff und Wasserdampf wird dazu über das Festbett eines Prereformierungskatalysators bei z. B. 530 °C geleitet. Im Prereformer werden höhere Kohlenwasserstoffe, vorliegend überwiegend C₄-Bausteine, in C₂- und C₁-Bausteine gespalten. Im Katalysatorbett tritt aufgrund der endothermen Reaktion eine Temperaturerniedrigung von z. B. 60 bis 70 °C auf. Das austretende Gas wird anschließend wieder auf die erforderliche Reformer-Eintrittstemperatur gebracht.

Der Prereformierungsschritt kann in analoger Weise durchgeführt werden, wie z. B. in H. Jockel, B. Triebskorn: "Gasynthan process for SNG", Hydrocarbon processing, Januar 1973, S. 93-98 beschrieben. Ein geeigneter Prereformierungskatalysator ist ein geträgerter Nickeloxid-Katalysator, wie er von der BASF AG, Ludwigshafen, unter der Bezeichnung G 1-80 vertrieben wird.

### Partielle Oxidation

Durch Umsetzung von Kohlenwasserstoffen mit unterstöchiometrischen Mengen an Sauerstoff kann ebenfalls Synthesegas hergestellt werden. Die Synthesegasherstellung durch partielle Oxidation kann durch folgende Summenformel beschrieben werden:

CHₓ + 1/2 O₂ → CO + x/2 H₂

Mit einer typischen Summenformel des Buten-abgereicherten C₄-Kohlenwasserstoffstroms von C₄H_{9,62} ergibt sich

C₄H_{9,62} + 2 O₂ → 4 CO + 4,81 H₂

Das molare H₂/CO-Verhältnis des erhaltenen Synthesegases beträgt 1,2:1. Synthesegas dieser Zusammensetzung ist gut geeignet zur Durchführung von Hydroformylierungen ohne Hydrierung. Für die Hydroformylierung und Hydrierung sind höhere H₂/CO-Verhältnisse erforderlich. Diese können durch Konvertierung eingestellt werden, wie weiter unten erläutert ist.

Zur Durchführung der partiellen Oxidation werden in der Regel die Kohlenwasserstoffe, Sauerstoff und gegebenenfalls Wasserdampf getrennt voneinander vorerhitzt und durch einen oder mehrere Brenner in den oberen Teil eines Reaktors eingeführt. Die Brenner gestatten eine rasche und innige Durchmischung der Reaktionsteilnehmer. Die vorerwärmten Einsatzprodukte reagieren ohne Katalysator bei etwa 30 bis 80 bar und 1200 bis 1500 °C im Verbrennungsteil des Reaktors. Die erzeugte Wärme dient der Dampfspaltung der Kohlenwasserstoffe. Das den Reaktor verlassende Gas wird entweder direkt durch Abschrecken mit Wasser oder indirekt durch Wärmeübertragung gekühlt. Aus einem kleinen Teil der Kohlenwasserstoffe entsteht meist Ruß. Dieser wird aus dem Synthesegas in der Regel durch Waschen mit H₂O entfernt.

Geeignete Anlagen zur Synthesegasherstellung durch partielle Oxidation sind z. B. in Max Appl, loc. cit., S. 106ff, beschrieben.

Die obigen Ausführungen zum Steamreforming bzw. zur partiellen Oxidation gelten entsprechend für den Einsatz des zweiten Buten-abgereicherten C₄-Kohlenwasserstoffstroms gemäß dem zweiten Aspekt der Erfindung.

### Synthesegas-Nachbehandlung

Ein gewünschtes Kohlenmonoxid/Wasserstoff-Verhältnis in einem Synthesegas kann durch sogenannte Konvertierung an geeigneten Katalysatoren in Gegenwart von Wasserdampf eingestellt werden. Die Konvertierung kann insbesondere zur Erhöhung des molaren H₂/CO-Verhältnisses in einem durch partielle Oxidation hergestellten Synthesegas herangezogen werden. Die zugrundeliegende Reaktion zwischen Kohlenmonoxid und Wasserdampf zu Kohlendioxid und Wasserstoff ist eine Gleichgewichtsreaktion:

CO + H₂O ⇄ CO₂ + H₂

Die Reaktion ist exotherm, daher verschieben tiefere Temperaturen das Gleichgewicht zur rechten Seite. Die Konvertierung kann ein- oder mehrstufig, z. B. bei 350 bis 570 °C und bei Normaldruck oder erhöhtem Druck in Gegenwart von Katalysatoren erfolgen. Geeignete Konvertierungskatalysatoren bestehen in der Regel aus Fe-Cr-Oxidgemischen. Mit ihnen lässt sich der CO-Gehalt, falls gewünscht, auf etwa 3 bis 4 Vol.-% absenken. Auf diese Weise kann weitgehend reiner Wasserstoff hergestellt werden, der z. B. für die Hydrierschritte der erfindungsgemäßen Verfahren geeignet ist.

Die Entfernung von Kohlendioxid kann durch Auswaschen mit geeigneten Lösungsmitteln erfolgen. Geeignete Verfahren sind z. B. die Druckwasser- und Kaliumcarbonat-Wäsche. Weitere zweckmäßige Verfahren sind Gaswäschen mit Monoethanolamin und Diethanolamin (vergleiche Ullmann's Encyclopedia of Industrial Chemistry, 5. Aufl., Band A12, S. 197).

Eine Übersicht über geeignete Verfahren zur Entfernung von Kohlendioxid findet sich ferner in Max Appl, loc. cit., S. 106ff.

### Hydroformylierung

Die Hydroformylierung oder Oxo-Synthese dient der Herstellung von Aldehyden aus Olefinen und Synthesegas, d. h. einem Gemisch von Kohlenmonoxid und Wasserstoff. Die erhaltenen Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -Komplexe eingesetzt, die ummodifiziert oder z. B. mit amin- oder phosphinhaltigen Verbindungen modifiziert sein können. Eine zusammenfassende Darstellung der großtechnisch ausgeübten Verfahren findet sich bei J. Falbe, "New Synthesis with Carbon Monoxide", Springer-Verlag 1980, Seite 162ff.

### Octen-Hydroformylierung

Während kurzkettige Olefine gegenwärtig überwiegend mit Ligandenmodifizierten Rhodiumcarbonylen als Katalysator hydroformyliert werden, behauptet bei den längerkettigen Olefine, z. B. Octene, Kobalt als katalytisch wirksames Zentralatom seine dominierende Stellung. Dies liegt zum einen an der hohen katalytischen Aktivität des Kobaltcarbonylkatalysators unabhängig von der Lage der olefinischen Doppelbindungen, der Verzweigungsstruktur und der Reinheit des umzusetzenden Olefins. Zum anderen kann der Kobaltkatalysator von den Hydroformylierungsprodukten vergleichsweise leicht abgetrennt und in die Hydroformylierungsreaktion zurückgeführt werden. Bei einem besonders zweckmäßigen Verfahren zur Hydroformylierung von Octenen geht man so vor, dass
a) eine wässrige Kobalt(II)-salzlösung mit Wasserstoff und Kohlenmonoxid unter Bildung eines hydroformylierungsaktiven Kobaltkatalysators innig in Kontakt gebracht wird; die den Kobaltkatalysator enthaltende wässrige Phase in wenigstens einer Reaktionszone mit den Octenen sowie Wasserstoff und Kohlenmonoxid innig in Kontakt gebracht wird, wobei der Kobaltkatalysator in die organische Phase extrahiert wird und die Octene hydroformyliert werden,
b) der Austrag aus der Reaktionszone in Gegenwart von saurer wässriger Kobalt(II)-salzlösung mit Sauerstoff behandelt wird, wobei der Kobaltkatalysator unter Bildung von Kobalt(II)-salzen zersetzt wird und diese in die wässrige Phase zurückextrahiert werden; und die Phasen anschließend getrennt werden;
c) die wässrige Kobalt(II)-salzlösung zurück in Schritt a) geführt wird.

Als Kobalt(II)-salze sind insbesondere Kobaltcarboxylate, wie Kobalt(II)-formiat, Kobalt(II)-acetat oder Kobaltethylhexanoat sowie Kobaltacetylacetonat geeignet. Die Katalysatorbildung kann gleichzeitig mit der Katalysatorextraktion und Hydroformylierung in einem Schritt in der Reaktionszone des Hydroformylierungsreaktors oder in einem vorgelagerten Schritt (Vorcarbonylierung) erfolgen. Die Vorcarbonylierung kann mit Vorteil gemäß der Beschreibung DE-OS 2 139 630 erfolgen. Die so erhaltene, Kobalt(II)-salze und Kobaltkatalysator enthaltende wässrige Lösung wird dann zusammen mit den zu hydroformylierenden Octenen sowie Wasserstoff und Kohlenmonoxid in die Reaktionszone geleitet. In vielen Fällen ist es jedoch bevorzugt, dass die Bildung des Kobaltkatalysators, die Extraktion des Kobaltkatalysators in die organische Phase und die Hydroformylierung in einem Schritt erfolgen, in dem die wässrige Kobalt(II)-salzlösung, die Olefine in der Reaktionszone unter Hydroformylierungsbedingungen innig in Kontakt gebracht werden. Die Ausgangsstoffe werden dabei so in die Reaktionszone eingebracht, dass eine gute Phasenvermischung erfolgt und eine möglichst hohe Phasenaustauschfläche erzeugt wird. Hierzu sind insbesondere Mischdüsen für Mehrphasensysteme geeignet.

Der Reaktionsaustrag wird nach Verlassen der Reaktionszone entspannt und in die Entkobaltungsstufe geleitet. In der Entkobaltungsstufe wird der Reaktionsaustrag in Gegenwart von wässriger, schwach saurer Kobalt(II)-salzlösung mit Luft oder Sauerstoff von Kobaltcarbonylkomplexen befreit. Bei der Entkobaltung wird der hydroformylierungsaktive Kobaltkatalysator unter Bildung von Kobalt(II)-salzen zersetzt. Die Kobalt(II)-salze werden in die wässrige Phase zurückextrahiert. Die wässrige Kobalt(II)-salzlösung kann anschließend in die Reaktionszone bzw. Katalysatorbildungsstufe zurückgeführt werden.

Das rohe Hydroformylierungsprodukt kann unmittelbar der Hydrierung zugeleitet werden; alternativ können die reinen C₉-Aldehyde nach bekannten Verfahren, z. B. durch Destillation, isoliert werden.

### Buten-Hydroformylierung

Der zweite Aspekt der Erfindung sieht die Hydroformylierung einer Teilmenge der im eingesetzten C₄-Kohlenwasserstoffstrom enthaltenen Butene vor, bevor der dabei erhaltene Buten-abgereicherte C₄-Kohlenwasserstoffstrom (vorliegend zur Unterscheidung von anderen in den erfindungsgemäßen Verfahren auftretenden Buten-abgereicherten C₄-Kohlenwasserstoffströmen auch "erster Buten-abgereicherter C₄-Kohlenwasserstoffstrom" genannt) der Butendimerisierung zugeführt wird.

Die Hydroformylierung eines 1-Buten, 2-Buten und gegebenenfalls Isobuten enthaltenden Kohlenwasserstoffstroms liefert C₅-Aldehyde, d. h. n-Valeraldehyd, 2-Methylbutanal und gegebenenfalls 2,2-Dimethylpropanal. Die Buten-Hydroformylierung erfolgt vorzugsweise in Gegenwart eines Rhodium-Komplexkatalysators in Verbindung mit einem Triorganophosphinliganden. Bei dem Triorganophosphinliganden kann es sich um ein Trialkylphosphin, wie Tributylphosphin, ein Alkyldiarylphosphin, wie Butyldiphenylphosphin, oder ein Aryldialkylphosphin, wie Phenyldibutylphosphin handeln. Besonders bevorzugt sind jedoch Triarylphosphinliganden, wie Triphenylphosphin, Tri-p-tolylphosphin, Trinaphthylphosphin, Phenyldinaphthylphosphin, Diphenylnaphthylphosphin, Tri(p-methoxyphenyl)phosphin, Tri(p-cyanophenyl)phosphin, Tri(p-nitrophenyl)phosphin, p-N,N-dimethylaminophenylbisphenylphosphin und dergleichen. Triphenylphosphin ist am meisten bevorzugt.

Die Buten-Hydroformylierung wird vorzugsweise unter Bedingungen durchgeführt, unter denen die Umsetzung von 1-Buten rasch erfolgt, während die Hydroformylierung von 2-Buten sowie Isobuten langsam erfolgt. Auf diese Weise kann man erreichen, dass bei der Hydroformylierung im Wesentlichen lediglich 1-Buten zu n-Valeraldehyd und 2-Methylbutanal umgewandelt wird, wohingegen das 2-Buten und gegebenenfalls Isobuten im Wesentlichen unverändert zurückisoliert werden. Somit wird ein Buten-abgereicherter C₄-Kohlenwasserstoffstrom erhalten, dessen Gehalt an 1-Buten gegenüber dem eingesetzten C₄-Kohlenwasserstoffstrom vermindert ist und der im Wesentlichen die ursprünglichen Mengen an 2-Buten und Isobuten des eingesetzten C₄-Kohlenwasserstoffstroms enthält. Das Verhältnis von n-Valeraldehyd zu 2-Methylbutanal in den erhaltenen C₅-Aldehyden beträgt vorzugsweise wenigstens 4:1, insbesondere wenigstens 8:1.

Die bevorzugte Hydroformylierung von 1-Buten gegenüber 2-Buten und Isobuten kann durch Verwendung eines großen Überschusses an Triorganophosphorliganden und durch sorgfältige Steuerung der Temperaturen der Partialdrücke der Reaktanden und/oder Produkte erreicht werden. So wird der Triorganophosphinligand vorzugsweise in einer Menge von wenigstens 100 Mol pro Grammatom Rhodium eingesetzt. Die Temperatur liegt vorzugsweise im Bereich von 80 bis 130 °C, der Gesamtdruck beträgt vorzugsweise nicht mehr als 5000 kPa, wobei der Partialdruck von Kohlenmonoxid weniger als 150 kPa und der Partialdruck von Wasserstoff im Bereich von 100 bis 800 kPa gehalten wird. Ein geeignetes Hydroformylierungsverfahren, bei dem ein Gemisch von Butenen eingesetzt wird, ist in der EP 0 016 286 beschrieben.

### Hydrierung

Für die Hydrierung der C₉-Aldehyde zu den C₉-Alkoholen sind prinzipiell auch die Katalysatoren der Hydroformylierung zumeist bei höherer Temperatur geeignet; im Allgemeinen werden jedoch selektivere Hydrierkatalysatoren vorgezogen, die in einer separaten Hydrierstufe eingesetzt werden. Geeignete Hydrierkatalysatoren sind im Allgemeinen Übergangsmetalle, wie z. B. Cr, Mo, W, Fe, Rh, Co, Ni, Pd, Rt, Ru usw. oder deren Mischungen, die zur Erhöhung der Aktivität und Stabilität auf Trägern, wie z. B. Aktivkohle, Aluminiumoxid, Kieselgur usw. aufgebracht werden können. Zur Erhöhung der katalytischen Aktivität können Fe, Co und bevorzugt Ni, auch in Form der Raney-Katalysatoren, als Metallschwamm mit einer sehr großen Oberfläche verwendet werden. Die Hydrierung der C₉-Aldehyde erfolgt in Abhängigkeit von der Aktivität des Katalysators, vorzugsweise bei erhöhten Temperaturen und erhöhtem Druck. Vorzugsweise liegt die Hydriertemperatur bei etwa 80 bis 250 °C, bevorzugt liegt der Druck bei etwa 50 bis 350 bar.

Das rohe Hydrierungsprodukt kann nach üblichen Verfahren, z. B. durch Destillation, zu den C₉-Alkoholen aufgearbeitet werden.

Der zur Hydrierung benötigte Wasserstoff stammt vorzugsweise zumindest teilweise aus dem durch Steamreforming oder partieller Oxidation des Buten-abgereicherten C₄-Kohlenwasserstoffstroms erhaltenen Synthesegas. Der Wasserstoff kann durch an sich bekannte Verfahren, wie Druckwechseladsorption (Pressure Swing Adsorption, PSA) oder Tieftemperaturdestillation gewonnen werden (vergleiche Max Appl, loc. cit., S. 108ff).

### Aldolkondensation

Zwei Moleküle C₅-Aldehyd können zu α,β-ungesättigten C₁₀-Aldehyden, wie insbesondere 2-Propyl-2-heptenal und 2-Propyl-4-methyl-2-hexenal, kondensiert werden. Die Aldolkondensation erfolgt auf an sich bekannte Weise z. B. durch Einwirkung einer wässrigen Base, wie Natronlauge oder Kalilauge. Alternativ kann auch ein heterogener basischer Katalysator, wie Magnesium- und/oder Aluminiumoxid, verwendet werden (vgl. z. B. die EP-A 792 862).

Das Produkt der Aldolkondensation wird dann mit Wasserstoff katalytisch zu C₁₀-Alkoholen, wie insbesondere 2-Propylheptanol und 2-Propyl-4-methylhexanol, hydriert. Zur Hydrierung gelten die weiter oben gemachten Ausführungen zur Hydrierung der C₉-Aldehyde entsprechend.

Die Erfindung wird nun durch die folgenden Beispiele, die zum Teil auf Simulationsrechnungen beruhen, näher veranschaulicht. Zur Durchführung der Simulationsrechnungen wurde das ASPENPLUS-Simulationsprogramm der Fa. ASPEN Tech verwendet. Mittels dieses Simulationsprogramms wurde das thermodynamische Gleichgewicht berechnet, das sich beim Steamreforming bzw. der partiellen Oxidation eines gegebenen Kohlenwasserstoffstroms unter bestimmten Bedingungen einstellt. Mit Katalysatoren, wie sie in der betrieblichen Praxis verwendet werden, wird das thermodynamische Gleichgewicht in der Regel annähernd erreicht. Bei der Simulation wurden Verfahrenstemperaturen und -drücke zugrunde gelegt, wie sie üblicherweise beim Steamreforming bzw. der partiellen Oxidation von Kohlenwasserstoffen angewendet werden.

### Beispiel 1

Ein C₄-Kohlenwasserstoffstrom, der 88 Mol-% Buten und 12 Mol-% Butan enthält, wird in einem adiabatischen Reaktor (Länge: 4 m, Durchmesser: 80 cm) bei 20 bar und 80 °C über ein Katalysatorbett geleitet. Der Katalysator war gemäß DE 4 339 713 in Form von Tabletten mit den Abmessungen 5 x 5 mm hergestellt worden (Zusammensetzung in Mol-% Aktivkomponenten: NiO 50 Mol-%, TiO₂ 12,5 Mol-%, SiO₂ 33,5 Mol-%, Al₂O₃ 4 Mol-%).

Vom Reaktoraustrag wurden die gebildeten Octene und höheren Butenoligomere abgetrennt. Das dabei erhaltene Buten-abgereicherte C₄-Kohlenwasserstoffgemisch enthielt 80,5 Mol-% Butan und 19,5 Mol-% Buten, entsprechend einem Buten-Umsatz von 95 %.

In einer Simulationsrechnung wurde die zu erwartende Synthesegas-zusammensetzung beim Steamreforming dieses Buten-abgereicherten C₄-Kohlenwasserstoffstroms errechnet. Es wurden folgende Parameter verwendet:
S/C = 2,5
(S/C = Mol des zum Steamreforming eingesetzten Dampfes pro Mol des im C₄-Einsatzgemischs enthaltenen C)
Reformer-Austrittstemperatur = 880 °C
CO₂-Rückführung = 100 %
Druck = 20 bar absolut

Kohlenwasserstoffzulauf:

| | |
|---|---|
| Zusammensetzung: | 80,5 Mol-% Butan 19,5 Mol-% Buten |
| Massenstrom: | 57 730 kg/h |
| Eintrittstemperatur: | 500 °C |

Wasserdampf:

| | |
|---|---|
| Massenstrom: | 180 152 kg/h |
| Eintrittstemperatur: | 500 °C |

Rückgeführtes CO₂:

| | |
|---|---|
| Massenstrom: | 107 833 kg/h |

Die Ergebnisse der Simulationsrechnung sind wie folgt:

Reformeraustritt:
Druck = 20 bar Absoluttemperatur = 880 °C

| | |
|---|---|
| Massenstrom: | 345 716 kg/h |
| Zusammensetzung: | CO 18,0 Mol-% CO₂ 11,8 Mol-% CH₄ 1,4 Mol-% H₂ 38,4 Mol-% H₂O 30,4 Mol-% Butan 0,0 Mol-% Buten 0,0 Mol-% |

Synthesegas nach CO₂-Wäsche:

| | |
|---|---|
| Massenstrom: | 124 629 kg/h |
| Zusammensetzung: | CO 31,1 Mol-% CO₂ 0,0 Mol-% CH₄ 2,4 Mol-% H₂ 66,5 Mol-% H₂O 0,0 Mol-% Butan 0,0 Mol-% Buten 0,0 Mol-% |

Das molare H₂/CO-Verhältnis ergibt sich zu 2,14. Ein Synthesegas dieser Zusammensetzung ist gut geeignet zur Hydroformylierung und Hydrierung der gemäß obiger Beschreibung erhaltenen Olefine nach üblichen Verfahren. Der CH₄-Gehalt des Synthesegases von 2,4 Mol-% ist nicht störend bei der Hydroformylierung.

### Beispiel 2

Beispiel 1 wurde wiederholt, wobei jedoch die Simulation für eine partielle Oxidation des Buten-abgereicherten C₄-Kohlenwasserstoffstroms durchgeführt wurde. Es wurden folgende Parameter zugrundegelegt: Temperatur 1200 °C, Druck 50 bar, keine CO₂-Rückführung.

Kohlenwasserstoffzulauf:

| | |
|---|---|
| Zusammensetzung: | 80,5 Mol-% Butan 19,5 Mol-% Buten |
| Massenstrom: | 57 730 kg/h |
| Temperatur: | 300 °C |

Wasserdampf:

| | |
|---|---|
| Massenstrom: | 36 200 kg/h |
| Temperatur: | 270 °C |

Sauerstoff:

| | |
|---|---|
| Massenstrom: | 46 703 kg/h |
| Temperatur: | 25 °C |

Bei der Simulation wurden folgende Ergebnisse erhalten:

Reaktoraustritt:

| | |
|---|---|
| Zusammensetzung: | 32,7 Mol-% CO 4,2 Mol-% CO₂ 0,35 Mol-% CH₄, 47,5 Mol-% H₂ 15,2 Mol-% H₂O 0,0 Mol-% Butan 0,0 Mol-% Buten 0,0 Mol-% O₂ |
| Massenstrom: | 158 635 kg/h |
| Temperatur: | 1 200 °C |
| Druck: | 50 bar |

Das molare H₂/CO-Verhältnis beträgt 1,455.

Es wurde außerdem die Synthesegas-Zusammensetzung des obigen Gases nach Hochtemperatur-Konvertierung simuliert. Bei der Simulation ging man davon aus, dass 91 646 kg/h des oben beschriebenen, am Reaktoraustritt austretenden Gases im Bypass an der Hochtemperatur-Konvertierung vorbeigeleitet wurden, der Rest der Hochtemperatur-Konvertierung unterzogen wurde. Bei der Simulation wurde folgendes Ergebnis erhalten:

Synthesegas nach Hochtemperatur-Konvertierung:

| | |
|---|---|
| Zusammensetzung: | 28,1 Mol-% CO 8,8 Mol-% CO₂ 0,35 Mol-% CH₄ 52,0 Mol-% H₂ 10,7 Mol-% H₂O 0,0 Mol-% Butan 0,0 Mol-% Buten 0,0 Mol-% O₂ |

Das molare H₂/CO-Verhältnis des Synthesegases nach Hochtemperatur-Konvertierung beträgt 1,85. Durch Variation des Bypasses sind andere H₂/CO-Verhältnisse zu erreichen, wodurch die Synthesegaszusammensetzung optimal an die zur Hydroformylierung und/oder Hydrierung der gemäß obiger Beschreibung erhaltenen Olefine angepasst werden kann.

## Patentansprüche

1. Verfahren zur Herstellung von C₉-Alkoholen, bei dem man
a) einen Buten und Butan enthaltenden C₄-Kohlenwasserstoffstrom bereitstellt;
b) den C₄-Kohlenwasserstoffstrom einer Oligomerisierung an einem Olefin-Oligomerisierungskatalysator unterwirft und das erhaltene Reaktionsgemisch auftrennt, wobei ein Octene enthaltender Strom und ein Buten-abgereicherter C₄-Kohlenwasserstoffstrom erhalten werden;
c) den Buten-abgereicherten C₄-Kohlenwasserstoffstrom einem Steamreforming oder einer partiellen Oxidation unterwirft, wobei Kohlenmonoxid und Wasserstoff erhalten werden;
d) den Octene enthaltenden Strom in Gegenwart eines Hydroformylierungskatalysators mit Kohlenmonoxid und Wasserstoff zu C₉-Aldehyden hydroformyliert, wobei das eingesetzte Kohlenmonoxid und der eingesetzte Wasserstoff zu mehr als 80% aus dem Schritt c) stammen; und
e) die C₉-Aldehyde mit Wasserstoff katalytisch hydriert, wobei der in Schritt e) eingesetzte Wasserstoff zu mehr als 80% aus Schritt c) stammt.

2. Verfahren nach Anspruch 1, bei dem der Buten-abgereicherte C₄-Kohlenwasserstoffstrom vor dem Steamreforming ganz oder teilweise hydriert wird.

3. Verfahren nach einem der vorhergehenden Ansprüche, bei dem das Steamreforming die Verwendung eines Prereformers umfasst.

4. Verfahren nach einem der vorhergehenden Ansprüche, bei dem das durch Steamreforming oder partielle Oxidation des Buten-abgereicherten C₄-Kohlenwasserstoffstroms erhaltene Gemisch von Kohlenmonoxid und Wasserstoff einer Hochtemperatur-Konvertierung unterworfen wird.

5. Verfahren nach einem der vorhergehenden Ansprüche, bei dem der C₄-Kohlenwasserstoffstrom 50 bis 99 Mol-% Buten enthält.

6. Verfahren nach einem der vorhergehenden Ansprüche, bei dem der Buten-abgereicherte C₄-Kohlenwasserstoffstrom 5 bis 70 Mol-% Buten enthält.

7. Verfahren nach einem der vorhergehenden Ansprüche, bei dem der Olefin-Oligomerisierungskatalysator ein heterogener, Nickel enthaltender Katalysator ist.

8. Verfahren zur integrierten Herstellung von C₉-Alkoholen und C₁₀-Alkoholen, bei dem man
a) einen Buten und Butan enthaltenden C₄-Kohlenwasserstoffstrom bereitstellt;
b) den C₄-Kohlenwasserstoffstrom in Gegenwart eines Hydroformylierungskatalysators mit Kohlenmonoxid und Wasserstoff zu C₅-Aldehyden hydroformyliert, wobei ein erster Buten-abgereicherter C₄-Kohlenwasserstoffstrom erhalten wird;
c) die C₅-Aldehyde einer Aldolkondensation unterwirft; und
d) die Produkte der Aldolkondensation mit Wasserstoff katalytisch zu C₁₀-Alkoholen hydriert;
e) den ersten Buten-abgereicherten C₄-Kohlenwasserstoffstrom an einem Olefin-Oligomerisierungskatalysator einer Oligomerisierung unterwirft und das erhaltene Reaktionsgemisch auftrennt, wobei ein Octene enthaltender Strom und ein zweiter Buten-abgereicherter C₄-Kohlenwasserstoffstrom erhalten werden;
f) den Octen enthaltenden Strom in Gegenwart eines Hydroformylierungskatalysators mit Kohlenmonoxid und Wasserstoff zu C₉-Aldehyden hydroformyliert;
g) die C₉-Aldehyde mit Wasserstoff katalytisch hydriert;
h) den zweiten Buten-abgereicherten C₄-Kohlenwasserstoffstrom einem Steamreforming oder einer partiellen Oxidation unterwirft, wobei Kohlenmonoxid und Wasserstoff erhalten werden, welche zumindest teilweise in Schritt b) und Schritt f) zurückgeführt werden, wobei der in Schritt d) und Schritt g) eingesetzte Wasserstoff zu mehr als 80% aus Schnitt h) stammt.

9. Verfahren nach Anspruch 8, bei dem der zweite Buten-abgereicherte C₄-Kohlenwasserstoffstrom vor dem Steamreforming ganz oder teilweise hydriert wird.

10. Verfahren nach einem der Ansprüche 8 oder 9, bei dem das Steamreforming die Verwendung eines Prereformers umfasst.

11. Verfahren nach einem der Ansprüche 8 bis 10, bei dem das durch Steamreforming oder partielle Oxidation des zweiten Buten-abgereicherten C₄-Kohlenwasserstoffstroms erhaltene Gemisch von Kohlenmonoxid und Wasserstoff einer Hochtemperatur-Konvertierung unterworfen wird.

12. Verfahren nach einem der Ansprüche 8 bis 11, bei dem der C₄-Kohlenwasserstoff 50 bis 99 Mol-% Buten enthält.

13. Verfahren nach einem der Ansprüche 8 bis 12, bei dem der Olefin-Oligomerisierungskatalysator ein heterogener, Nickel enthaltender Katalysator ist.

## Claims

1. A process for preparing C₉-alcohols, which comprises
a) providing a C₄-hydrocarbon stream comprising butene and butane;
b) subjecting the C₄-hydrocarbon stream to oligomerization over an olefin oligomerization catalyst and fractionating the resulting reaction mixture to give an octene-comprising stream and a butene-depleted C₄-hydrocarbon stream;
c) subjecting the butene-depleted C₄-hydrocarbon stream to steam reforming or partial oxidation to give carbon monoxide and hydrogen;
d) hydroformylating the octene-comprising stream by means of carbon monoxide and hydrogen in the presence of a hydroformylation catalyst to form C₉-aldehydes, where the carbon monoxide used and the hydrogen used originate to an extent of more than 80% from step c); and
e) catalytically hydrogenating the C₉-aldehydes by means of hydrogen, where the hydrogen used in step e) originates to an extent of more than 80% from step c).

2. The process according to claim 1, wherein the butene-depleted C₄-hydrocarbon stream is fully or partially hydrogenated prior to steam reforming.

3. The process according to either of the preceding claims, wherein the steam reforming includes the use of a prereformer.

4. The process according to any of the preceding claims, wherein the mixture of carbon monoxide and hydrogen obtained by steam reforming or partial oxidation of the butene-depleted C₄-hydrocarbon stream is subjected to high-temperature converting.

5. The process according to any of the preceding claims, wherein the C₄-hydrocarbon stream comprises from 50 to 99 mol% of butene.

6. The process according to any of the preceding claims, wherein the butene-depleted C₄-hydrocarbon stream comprises from 5 to 70 mol% of butene.

7. The process according to any of the preceding claims, wherein the olefin oligomerization catalyst is a heterogeneous, nickel-comprising catalyst.

8. A process for the integrated preparation of C₉-alcohols and C₁₀-alcohols, which comprises
a) providing a C₄-hydrocarbon stream comprising butene and butane;
b) hydroformylating the C₄-hydrocarbon stream by means of carbon monoxide and hydrogen to form C₅-aldehydes, so as to give a first butene-depleted C₄-hydrocarbon stream;
c) subjecting the C₅-aldehydes to an aldol condensation; and
d) catalytically hydrogenating the products of the aldol condensation by means of hydrogen to form C₁₀-alcohols;
e) subjecting the first butene-depleted C₄-hydrocarbon stream to oligomerization over an olefin oligomerization catalyst and fractionating the resulting reaction mixture to give an octene-comprising stream and a second butene-depleted C₄-hydrocarbon stream;
f) hydroformylating the octene-comprising stream by means of carbon monoxide and hydrogen in the presence of a hydroformylation catalyst to form C₉-aldehydes;
g) catalytically hydrogenating the C₉-aldehydes by means of hydrogen;
h) subjecting the second butene-depleted C₄-hydrocarbon stream to steam reforming or partial oxidation to give carbon monoxide and hydrogen which are recirculated at least in part to step b) and step f), where the hydrogen used in step d) and step g) originates to an extent of more than 80% from step h).

9. The process according to claim 8, wherein the second butene-depleted C₄-hydrocarbon stream is fully or partially hydrogenated prior to steam reforming.

10. The process according to either of claims 8 and 9, wherein the steam reforming includes the use of a prereformer.

11. The process according to any of claims 8 to 10, wherein the mixture of carbon monoxide and hydrogen obtained by steam reforming or partial oxidation of the second butene-depleted C₄-hydrocarbon stream is subjected to high-temperature converting.

12. The process according to any of claims 8 to 11, wherein the C₄-hydrocarbon comprises from 50 to 99 mol% of butene.

13. The process according to any of claims 8 to 12, wherein the olefin oligomerization catalyst is a heterogeneous, nickel-comprising catalyst.

## Revendications

1. Procédé de fabrication d'alcools en Cg, selon lequel
a) un courant d'hydrocarbures en C₄ contenant du butène et du butane est préparé ;
b) le courant d'hydrocarbures en C₄ est soumis à une oligomérisation sur un catalyseur d'oligomérisation d'oléfines et le mélange réactionnel obtenu est séparé, un courant contenant des octènes et un courant d'hydrocarbures en C₄ appauvri en butène étant obtenus ;
c) le courant d'hydrocarbures en C₄ appauvri en butène est soumis à un reformage à la vapeur ou à une oxydation partielle, du monoxyde de carbone et de l'hydrogène étant obtenus ;
d) le courant contenant des octènes est hydroformylé en présence d'un catalyseur d'hydroformylation avec du monoxyde de carbone et de l'hydrogène pour former des aldéhydes en Cg, le monoxyde de carbone utilisé et l'hydrogène utilisé étant issus à plus de 80 % de l'étape c) ; et
e) les aldéhydes en C₉ sont hydrogénés catalytiquement avec de l'hydrogène, l'hydrogène utilisé à l'étape e) étant issu à plus de 80 % de l'étape c).

2. Procédé selon la revendication 1, selon lequel le courant d'hydrocarbures en C₄ appauvri en butène est hydrogéné en totalité ou en partie avant le reformage à la vapeur.

3. Procédé selon l'une quelconque des revendications précédentes, selon lequel le reformage à la vapeur comprend l'utilisation d'un pré-reformeur.

4. Procédé selon l'une quelconque des revendications précédentes, selon lequel le mélange de monoxyde de carbone et d'hydrogène obtenu par reformage à la vapeur ou oxydation partielle du courant d'hydrocarbures en C₄ appauvri en butène est soumis à une conversion à température élevée.

5. Procédé selon l'une quelconque des revendications précédentes, selon lequel le courant d'hydrocarbures en C₄ contient 50 à 99 % en moles de butène.

6. Procédé selon l'une quelconque des revendications précédentes, selon lequel le courant d'hydrocarbures en C₄ appauvri en butène contient 5 à 70 % en moles de butène.

7. Procédé selon l'une quelconque des revendications précédentes, selon lequel le catalyseur d'oligomérisation d'oléfines est un catalyseur hétérogène contenant du nickel.

8. Procédé de fabrication intégrée d'alcools en C₉ et d'alcools en C₁₀, selon lequel
a) un courant d'hydrocarbures en C₄ contenant du butène et du butane est préparé ;
b) le courant d'hydrocarbures en C₄ est hydroformylé en présence d'un catalyseur d'hydroformylation avec du monoxyde de carbone et de l'hydrogène pour former des aldéhydes en C₅, un premier courant d'hydrocarbures en C₄ appauvri en butène étant obtenu ;
c) les aldéhydes en C₅ sont soumis à une condensation d'aldol ; et
d) les produits de la condensation d'aldol sont hydrogénés catalytiquement avec de l'hydrogène pour former des alcools en C₁₀ ;
e) le premier courant d'hydrocarbures en C₄ appauvri en butène est soumis à une oligomérisation sur un catalyseur d'oligomérisation d'oléfines et le mélange réactionnel obtenu est séparé, un courant contenant des octènes et un second courant d'hydrocarbures en C₄ appauvri en butène étant obtenus ;
f) le courant contenant de l'octène est hydroformylé en présence d'un catalyseur d'hydroformylation avec du monoxyde de carbone et de l'hydrogène pour former des aldéhydes en C₉ ;
g) les aldéhydes en Cg sont hydrogénés catalytiquement avec de l'hydrogène ;
h) le second courant d'hydrocarbures en C₄ appauvri en butène est soumis à un reformage à la vapeur ou une oxydation partielle, du monoxyde de carbone et de l'hydrogène étant obtenus, qui sont recyclés au moins en partie dans l'étape b) et l'étape f), l'hydrogène utilisé dans l'étape d) et l'étape g) étant issu à plus 80 % de l'étape h).

9. Procédé selon la revendication 8, selon lequel le second courant d'hydrocarbures en C₄ appauvri en butène est hydrogéné en totalité ou en partie avant le reformage à la vapeur.

10. Procédé selon l'une quelconque des revendications 8 ou 9, selon lequel le reformage à la vapeur comprend l'utilisation d'un pré-reformeur.

11. Procédé selon l'une quelconque des revendications 8 à 10, selon lequel le mélange de monoxyde de carbone et d'hydrogène obtenu par reformage à la vapeur ou oxydation partielle du second courant d'hydrocarbures en C₄ appauvri en butène est soumis à une conversion à température élevée.

12. Procédé selon l'une quelconque des revendications 8 à 11, selon lequel l'hydrocarbure en C₄ contient 50 à 99 % en moles de butène.

13. Procédé selon l'une quelconque des revendications 8 à 12, selon lequel le catalyseur d'oligomérisation d'oléfines est un catalyseur hétérogène contenant du nickel.
